(19) Europäisches Patentamt
European Patent Office
Office européen des brevets

(11) Numéro de publication: **0 360 676 A1**

# (12) DEMANDE DE BREVET EUROPEEN

(21) Numéro de dépôt: **89402548.5**

(51) Int. Cl.5: **C 12 Q 1/68**

(22) Date de dépôt: **18.09.89**

(30) Priorité: **20.09.88 FR 8812284**

(43) Date de publication de la demande: **28.03.90 Bulletin 90/13**

(84) Etats contractants désignés: **CH DE ES FR GB LI NL SE**

(71) Demandeur: **COMMISSARIAT A L'ENERGIE ATOMIQUE**
**31/33, rue de la Fédération**
**F-75015 Paris (FR)**

(72) Inventeur: **Haan, Serge**
**18 Domaine de la Butte à la Reine**
**F-91120 Palaiseau (FR)**

(74) Mandataire: **Lhoste, Catherine et al**
**BREVATOME 25, rue de Ponthieu**
**F-75008 Paris (FR)**

## (54) procédé et installation de séquençage de l'ADN.

(57) Le procédé selon l'invention comporte essentiellement une étape (2) de fragmentation de l'ADN, une étape (4) de réactions chimiques spécifiques des bases constituant l'ADN pour former des familles de chaînes de tailles différentes dont l'une des extrémités est constituée d'une base spécifique, une étape (6) de marquage spécifique de ces chaînes avec des isotopes stables, une étape (10) de séparation des chaînes par électrophorèse, une étape d'atomisation et d'ionisation (12) des chaînes séparées, une étape (14) de sélection et de détection en temps réel des ions par spectrométrie de masse et une étape de traitement (16) des signaux détectés pour reconstituer l'ADN de départ.

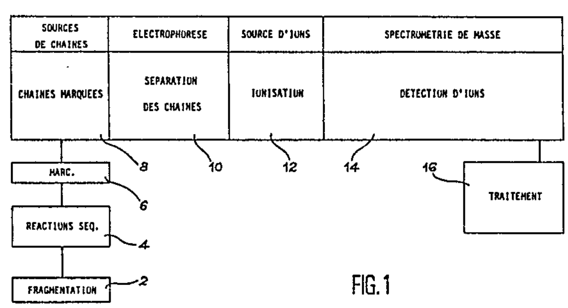


FIG. 1

EP 0 360 676 A1

## Description

### PROCEDE ET INSTALLATION DE SEQUENCAGE DE L'ADN.

L'invention se rapporte à un procédé et une installation de séquençage de molécules biologiques complexes telles que l'ADN (acide désoxyribonucléique), l'ARN (acide ribonucléique), les protéines. Elle s'applique dans les domaines du génie génétique, de la biologie, de la pharmacologie, tant au stade de l'expérimentation, de la recherche fondamentale qu'industrielle.

Le séquençage qui en fait est l'identification des séquences ou de l'ordre des nucléotides ou des acides aminés des molécules complexes a pris une réelle importance dans le domaine de la biologie moléculaire et de la biotechnologie. En particulier, le séquençage d'un acide nucléique (ADN ou ARN) permet l'analyse des génomes animaux, végétaux et viraux dans des gènes particuliers. Puisque la fonction d'une molécule biologique est déterminée par sa structure, la définition de la structure d'un gène est cruciale pour la manipulation éventuelle de cette unité de base d'information.

Le procédé de l'invention s'applique de façon générale à toutes molécules constituées d'une succession de nucléotides ou d'acides aminés et plus spécialement à l'ADN. On rappelle qu'un nucléotide est formé par une base, un sucre et un phosphate.

Pour les acides nucléiques, les bases sont des bases azotées possédant une structure hétérocyclique du type purique ou pyrimidique ; elles sont au nombre de 4. Le sucre est le ribose pour l'ARN et le désoxyribose pour l'ADN.

Généralement, les nucléotides des acides nucléiques utilisés sont triphosphatés car c'est ce type de molécules qui fournit l'énergie nécessaire au fonctionnement des enzymes dans de nombreuses réactions biochi miques, en particulier celles intervenant dans la polymérisation des ADN.

L'ARN et l'ADN présentent trois bases communes : l'adénine (A) et la guanine (G) qui sont des purines et la cytosine (C) qui est une pyrimidine. L'autre base pyrimidique est la thymine (T) pour l'ADN et l'uracile (U) pour l'ARN.

Pour l'ADN, les quatre types de nucléotides concernés sont le désoxy adénosine triphosphate ou dATP, le désoxy guanine triphosphate ou dGTP, le désoxy cytosine triphosphate ou dCTP et le désoxy thymine triphosphate ou dTTP. On les note ci-après dxTP avec X = A, G, C ou T.

L'ADN contient l'information génétique complète nécessaire à la fabrication des protéines, éléments constitutifs de tout organisme vivant. En particulier, chaque succession de trois bases est le code, par l'intermédiaire des ARN messagers, d'un acide aminé qui entre dans la composition d'une protéine. Le séquençage a pour but d'obtenir l'ordre des bases constituant un brin d'ADN ou d'ARN.

Les méthodes de séquençage de l'ADN comportent essentiellement 5 étapes qui sont (a) la fragmentation du génome, (b) les réactions chimiques de séquençage pour former des chaînes de nucléotides, (c) le marquage des chaînes de nucléotides, (d) la séparation des chaînes marquées et (e) la détection.

Etape a

Un génome ou ensemble des secteurs de l'ADN est constitué par un grand nombre de paires de bases (13 x $10^6$ pour la levure et 3 x $10^9$ pour l'homme). Le génome, du fait de sa longueur, ne peut être directement soumis aux réactions de séquençage. L'ADN est scindé en fragments d'environ 1000 à 30000 bases qui sont ensuite multipliés par clonage. Par exemple, cette fragmentation peut être effectuée par l'utilisation d'enzymes de restriction qui coupent l'ADN au niveau de certaines séquences ou par l'utilisation des ultra-sons, comme exposé dans l'article "Current Approaches to DNA Sequencing" de J.C. Moores, Analytic Biochemistry 163, 1-8 (1987).

Etape b

Elle consiste à appliquer aux chaînes obtenues en (a) quatre séries de réactions chimiques spécifiques des quatre bases de l'ADN. Ces réactions conduisent à l'obtention de quatre familles de chaînes d'ADN dont l'une des extrémités est communément fixée et l'autre est constituée d'une base spécifique de l'une des quatre séries de réaction.

Deux méthodes sont communément utilisées pour obtenir les 4 familles de chaînes d'ADN ci-dessus. L'une, dévelopée par Maxam et Gilbert (Maxam A.M. et Gilbert W., Proc. Natl. Acad. Sci., U.S.A., vol. 74, N° 2, pp 560-564 de 1977, "A new method for sequencing DNA") fait appel à une procédure de dégradation chimique des chaînes et l'autre, introduite par Sanger (Sanger F. et al., Proc. Natl. Acad. Sci. USA 74, pp. 5453-5467 de Décembre 1977 "DNA sequencing with chain-terminating inhibitors") est une méthode enzymatique et emploie un procédé de terminaison de chaînes didésoxy.

Dans la méthode de Sanger, chacune des quatre réactions de séquençage met en jeu un grand nombre d'exemplaires d'ADN monocaténaire à séquencer ; un amorceur (ou en terminologie anglo-saxonne primer) qui est un oligo-nucléotide complémentaire de l'une des extrémités de l'ADN à séquencer ; les quatre nucléotides dXTP ; une enzyme qui permet la fabrication du brin complémentaire de l'ADN à séquencer, à partir des nucléotides et en commençant par l'amorceur et enfin un seul des quatre di-désoxy nucléotides appelé nucléotide terminal et noté ddXTP. Ce ddXTP est un nucléotide auquel il manque le groupement OH du carbone 3′ du désoxyribose.

L'amorceur se lie à l'ADN à séquencer par complémentarité puis l'enzyme synthétise le brin

complémentaire jusqu'à ce qu'elle incorpore le ddXTP arrêtant l'élongation de la chaîne. L'incorporation du nucléotide terminal peut se produire n'importe quand au cours de la synthèse ce qui se traduit, à la fin de l'opération de séquençage spécifique d'une base X donnée, par la présence d'une famille d'ADN représentant toutes les longueurs possibles depuis l'amorceur jusqu'à environ 400 nucléotides.

Appliquée en parallèle pour les quatre ddXTP, la série de quatre réactions conduit à un ensemble de quatre familles de chaînes d'ADN ; les chaînes de chaque famille étant représentatives d'une base X donnée sont ensuite déposées sur un gel d'électrophorèse.

Dans la méthode de Maxam et Gilbert, des chaînes d'ADN de dimensions adaptées et analogues à celles issues du procédé de Sanger sont produites par un traitement chimique d'ADN purifié. Cette opération permet de séquencer un ADN mono- ou bicaténaire marqué avec un phosphate radioactif à l'une de ses extrémités.

Pour chacune des quatre séries de réaction de séquençage, l'ADN est soumis à un jeu de deux réactions chimiques. la première réaction chimique modifie et retire la base de son sucre, la seconde coupe l'ADN en retirant le sucre ainsi exposé. On obtient ainsi des chaînes commençant à un endroit fixé et se terminant par une base connue. Les conditions de réaction sont ajustées de façon à produire des chaînes de toutes les longueurs possibles comportant typiquement de 1 à 400 nucléotides.

Etape c

Les chaînes obtenues en (b) sont marquées préalablement, ou au cours des réactions, avec des marqueurs radioactifs. Les marqueurs radioactifs utilisés sont le $^{32}P$ ou le $^{35}S$, le tritium, le $^{14}C$ qui sont des émetteurs de rayonnements$\beta$-.

Dans le cadre de la méthode de Sanger, il est possible de marquer l'amorceur, les dXTP ou les ddXTP.

Etape d

Les chaînes d'ADN marquées sont alors séparées suivant leur longueur, les plus petites migrant le plus vite et les plus grandes le plus lentement. Ce gel d'électrophorèse qui est en général un gel de polyacrylamide permet de différencier les molécules de longueur N et N+1 avec N variant de 1 à 400 environ.

L'électrophorèse dure de 4 à 12 heures suivant la longueur du gel qui est ensuite séché.

Etape e

Le gel est ensuite placé contre un film sensible au rayonnement béta. Les marqueurs radioactifs incorporés dans les chaînes d'ADN sont utilisés pour développer une image auto-radiographique de chaque tracé électrophorétique. L'auto-radiographie est développée après 12 heures ou plus d'exposition au rayonnement.

La position relative des bandes qui apparaissent sur l'image auto-radiographique, au sein des quatre colonnes correspondant aux quatre familles de réactions, permet la détermination de la séquence des bases de l'ADN de départ.

Après la lecture, l'examen et l'interprétation de l'auto-radiographie, la séquence est entrée dans la mémoire d'un ordinateur pour une analyse et un assemblage ultérieurs.

Ces deux méthodes de séquençage d'ADN précédemment décrites sont largement utilisées et comportent chacune plusieurs variantes. Bien que très performantes et efficaces, ces méthodes demandent une mise en oeuvre très complexe qui comporte de sérieux inconvénients. C'est en réalité un très gros travail délicat, qui doit être extrêmement bien standardisé pour que la source de connaissances et la comparaison des migrations électrophorétiques soient fiables.

Par ailleurs, la lecture des électrophorèses des chaînes radioactives se fait après auto-radiographie. Là aussi, une parfaite reproductibilité est de rigueur tant dans le développement de ces auto-radiogrammes que dans leur lecture.

En effet, l'image sur film d'une bande de gel radioactif est plus large que la bande elle-même et la comparaison des positions des bandes entre les quatre pistes différentes de l'électrophorèse (qui peuvent induire un comportement non uniforme quant aux mobilités des chaînes) peut limiter la résolution effectivement observée sur l'auto-radiogramme et donc réduire la longueur de la séquence qui peut être lue à partir des gels. Typiquement, de 200 à 400 bases peuvent être lues à partir d'un simple jeu de tracés.

De plus, les irrégularités survenant d'un tracé à un autre peuvent rendre difficile la lecture automatique des auto-radiogrammes par des techniques d'imagerie existantes (par exemple densitomètres à balayages) car l'oeil humain peut actuellement mieux discriminer ces irrégularités que ne le font les ordinateurs.

La nécessité d'un soin intensif quant à l'analyse "manuelle" des auto-radiogrammes demande donc du temps et est sujette à des erreurs.

Par ailleurs, l'utilisation de marqueurs radioactifs présente de sérieux inconvénients quant à la manipulation (système de protection complexe des opérateurs et décontamination périodique des installations), au stockage des produits durant l'analyse et au stockage des déchets en fin d'analyse

Enfin, il n'est pas possible d'observer les bandes au sein du gel pendant l'électrophorèse afin de contrôler l'opération en cours, mais il faut nècessairement terminer l'électrophorèse à un moment déterminé et attendre le développement de l'auto-radiogramme avant de pouvoir lire la séquence.

Aussi, plusieurs procédés ont déjà envisagé la détection en temps réel des chaînes d'ADN pendant leur migration au cours de l'électrophorèse.

Les uns s'intéressent par exemple à la détection du rayonnement $\beta^-$ émis par les chaînes marquées au $^{32}P$, les autres s'intéressent à la détection de l'émission de fluorescence induite par un faisceau laser.

Dans le premier cas, la détection en ligne (ou temps réel) des bandes radioactives au cours de l'électrophorèse peut permettre une certaine simplification, par une certaine réduction du nombre des opérations à effectuer, pour l'ensemble du procédé. Cependant, il faut tenir compte de la courte période de demi-vie du phosphore 32 et par conséquent de l'instabilité des réactifs de marquage radioactif. En outre, comme dans les méthodes plus traditionnelles de Sanger ou Maxam et Gilbert, il demeure que des problèmes sont posés par l'élimination et la manipulation des produits radioactifs.

En outre, le temps de passage des chaînes devant le détecteur étant très réduit, cela implique une plus faible efficacité de collection des rayonnements, ce qui peut entraîner des problèmes de sensibilité et de résolution.

La détection de l'émission de fluorescence induite par un faisceau laser a été proposée par Ansorge (voir DE-A-3 618 605 et l'article Journal of Biochemical and Biophysical Methods. 13(1986), pp 315-323, "A non-radioactif automated method for DNA sequence determination". Cette méthode utilise un seul fluorophore pour marquer l'amorceur (ou primer) dans le cadre de la méthode de Sanger.

Dans ce cas, les quatre séries de réactions de séquençage conduisent, comme décrit précédemment, à quatre familles de chaînes d'ADN déposées sur quatre pistes parallèles d'un gel d'électrophorèse, qui est traversé entièrement, suivant sa largeur, par un faisceau laser. Au sein du gel, les chaînes d'ADN marquées sont excitées les unes après les autres, dans l'ordre de la séquence, lorsqu'elles passent dans le faisceau laser, au cours de l'électrophorèse.

Pour chaque piste d'électrophorèse associée à une famille de réactions donnée, la lumière de fluorescence est collectée par une optique adaptée et conduite, après filtrage, à un détecteur de type photomultiplicateur.

Un avantage offert par cette technique est l'absence de toute partie mobile au sein de l'appareil de détection grâce aux détecteurs fixes qui permettent une détection continue sur les quatre pistes d'électrophorèse.

Toutefois, l'utilisation d'un seul fluorophore conduit inévitablement à l'utilisation de quatre pistes en parallèle, sur le gel d'électrophorèse, pour l'étude d'une seule séquence, ce qui limite les possibilités d'évolution sensible et de simplification ultérieures. Il en est de même lors de la détection en temps réel des chaînes d'ADN marquées par du phosphore 32.

Ces systèmes de détection en temps réel nécessitent aussi une grande fiabilité quant à la comparaison des mobilités électrophorétiques d'une piste à l'autre. Or, en pratique, plusieurs difficultés peuvent être rencontrées ; en particulier, la formation de gradient thermique et la présence d'impuretés au sein du gel peuvent induire des distorsions locales des chaînes au sein du gel, et par suite compromettre directement l'attribution des bases dans la séquence cherchée.

D'autres procédés de détection en temps réel proposent l'utilisation de plusieurs fluorophores afin d'identifier individuellement chaque famille de chaînes. Ce type de procédé présente l'avantage, dans son concept, d'offrir la possibilité de séparer des chaînes d'ADN issues des réactions de séquençage, sur une seule et même piste d'électrophorèse. Ces systèmes utilisent une série de quatre fluorophores, ce qui semble répondre aux divers problèmes soulignés précédemment. Toutefois, la mise en oeuvre spécifique de chacun de ces systèmes ne rend ces approches que partiellement réussies.

Un premier système a été proposé par L.M. Smith et al. (voir FR-A-2 558 262), dans le cadre des réactions de séquençage de Sanger. Dans cette technique, les réactions de séquençage doivent s'effectuer dans quatre tubes séparés, chacun comprenant un marqueur fluorescent différent lié au "primer". L'appareil de détection décrit par Smith et al. utilise une série de filtres interférentiels à bande étroite afin de sélectionner les longueurs d'onde spécifiques d'émission de chacun des fluorophores.

A partir de ce procédé relativement simple, le type de système qui en résulte comporte au demeurant certaines imperfections. En particulier, de sérieux problèmes sont engendrés par l'utilisation de molécules fluorescentes de charges et de tailles très différentes.

Cette situation se traduit par l'existence de perturbations au cours de l'électrophorèse dues aux différences de mobilités électrophorétiques des diverses chaînes marquées différemment. En effet, malgré l'élaboration conjointe d'un logiciel lourd et assez complexe de traitement et de correction des données, ces phénomènes peuvent entraîner certains recouvrements voire des inversions de chaînes au cours de l'électrophorèse, empêchant ou même faussant l'identification de certaines bases le long de la séquence.

Par ailleurs, cette technique requiert obligatoirement l'utilisation de quatre "primers" spécifiques marqués. Ceci entraîne, dans les cas où d'autres vecteurs sont employés, la nécessité d'entreprendre des processus longs et complexes de synthèse et de purification de quatre nouveaux "primers" fluorescents.

Un second système proposé par J.M. Prober et al. (voir EP-A-0 252 683) repose, toujours dans le cadre des réactions Sanger, sur l'utilisation de quatre fluorophores liés aux quatre ddXTP. Ce marquage différent des ddXTP procure un certain nombre d'avantages. En particulier, les quatre réactions de séquençage peuvent s'effectuer dans un même tube, un amorceur unique non marqué peut alors être utilisé et les produits de réaction peuvent être séparés au sein d'une même colonne d'électrophorèse.

Cependant, le fait de greffer au ddXTP des marqueurs extrinsèques, c'est-à-dire les fluorophores, qui sont des molécules assez complexes et très volumineuses (les fluorophores utilisés sont dans ce cas les dérivés de la fluorescéine), peut entraîner des décalages imprévisibles dans les mobilités de certaines chaînes au cours de l'électrophorèse et surtout restreindre fortement le choix d'enzymes de complémentarité ; ceci élimine la possibilité d'utilisation des enzymes courantes, comme par exemple le fragment de Klenow d'ADN polymérase.

L'invention a donc pour objet un procédé d'identification des séquences d'un ADN permettant de remédier à la plupart des inconvénients donnés précédemment. Des problèmes similaires apparaissant dans le séquençage de tout autre acide nucléique et, en particulier, dans les ARN, l'invention s'applique à tout type d'acide nucléique.

Aussi, l'invention a pour objet un procédé d'identification de la séquence des bases d'un acide nucléique comportant essentiellement :

a) une étape de production de fragments à sequencer,

b) une étape de réactions chimiques spécifiques desdites bases pour former des familles de chaînes de tailles différentes dont l'une des extrémités est constituée d'une base spécifique,

c) une étape de marquage spécifique desdites chaînes avec des isotopes ou petites molécules stables,

d) une étape de séparation des chaînes marquées,

e) une étape d'atomisation et d'ionisation de chaque chaîne marquée puis,

f) une étape de détection en temps réel des ions formés par spectrométrie de masse.

Le fonctionnement en temps réel est lié au fait que, dans l'invention, les ions sont détectés au fur et à mesure de leur formation.

Le procédé selon l'invention, grâce à l'utilisation de trois, quatre ou plusieurs isotopes ou petites molécules stables, c'est-à-dire non radioactives, donc non dangereuses pour les opérateurs, facilite considérablement les interventions humaines dans le procédé de séquençage. En outre, l'ionisation des chaînes marquées puis la détection de ces chaînes par un spectromètre ou analyseur de masse facilite considérablement la détection des chaînes marquées et donc le séquençage des acides nucléiques.

Le procédé de l'invention fonctionne en continu et ne comporte pas d'étape de radiographie selon l'art antérieur, longue et délicate.

En outre, l'ionisation et la spectrométrie de masse permettent l'utilisation de marqueurs liés de manière intrinsèque ou extrinsèque aux différents nucléotides, ce qui autorise l'application de ce procédé aux diverses méthodes chimiques de séquençage les plus répandues, et en particulier, celles de Sanger ou de Maxam-Gilbert relatives au séquençage de l'ADN.

Par petites molécules, il faut comprendre des composés chimiques pouvant être liés aux nucléotides et qui comportent au moins un élément marqueur identifiable par spectrométrie de masse, tel que $CF_2$, $Si(CH_3)_3$, en utilisant par exemple une technique décrite dans l'article Tetrahedron Letters, vol. 26, N° 40, pp. 4915-4918 (1985) C.D. Pein, D. Cech.

L'invention a aussi pour objet une installation d'identification de la séquence des bases d'un acide nucléique comprenant :

- une source de chaînes de nucléotides de tailles différentes, marquées par des isotopes ou petites molécules stables,
- des moyens de séparation de ces chaînes marquées,
- des moyens d'atomisation et d'ionisation des chaînes sortant des moyens de séparation,
- un spectromètre de masse pour sélectionner et détecter les ions et
- des moyens de traitement des signaux fournis par le spectromètre.

De façon avantageuse, on utilise comme moyen de séparation des chaînes marquées, un dispositif de chromatographie monopiste afin d'éliminer les erreurs de comparaison entre plusieurs pistes comme dans l'art antérieur.

Des problèmes similaires à ceux décrits précédemment pour le séquençage de l'ADN pouvant intervenir lors de l'identification de la séquence des acides aminés d'une protéine et de la détermination d'une protane particulière dans un mélange de protéines, l'invention a encore pour objet un procédé d'identification d'une protéine dans un mélange de protéines ou de ses acides aminés, comportant essentiellement :

- une étape de marquage spécifique desdits acides aminés avec des isotopes ou de petites molécules stables,
- une étape de séparation des protéines marquées,
- une étape d'atomisation et d'ionisation de chaque protéine marquée puis,
- une étape de détection en temps réel des ions formés par spectrométrie de masse.

L'invention a encore pour objet un procédé d'identification de la taille des fragments de molécules biologiques complexes, comprenant essentiellement :

- une étape de marquage des fragments,
- une étape de séparation selon leur taille, des fragments marqués spécifiquement et mêlés à des standards de taille,
- une étape d'atomisation et d'ionisation des fragments puis une détection en temps réel des ions par spectrométrie de masse.

D'autres caractéristiques et avantages de l'invention ressortiront de la description qui suit, donnée à titre illustratif et non limitatif aux dessins annexés, dans lesquels :

- la figure 1 représente un schéma d'ensemble des étapes de séquençage automatisé d'ADN selon l'invention,
- la figure 2 représente un schéma d'ensemble d'une installation automatisée pour le séquençage d'ADN conforme à l'invention,
- la figure 3 représente le type de données produites par un séquençage d'ADN selon l'invention.

La description qui va suivre se réfère au séquençage de l'ADN, bien que, comme on l'a dit précédemment,

l'invention s'applique à tout acide nucléique (ARN par exemple) ou toute protéine complexe.

La première étape du procédé, en référence à la figure 1, consiste à fragmenter un génome d'ADN (étape a), conformément à l'art antérieur cité. Cette étape de fragmentation porte la référence 2 sur la figure 1.

Les chaînes obtenues sont alors soumises aux réactions de séquençage (étape b) selon les méthodes usuelles si ce n'est que l'échelle peut être augmentée, en cas de besoin pour fournir une intensité de signal adéquate pour la détection de chaque famille de chaînes. Aucun nucléotide radio marqué n'a besoin d'être inclu dans les réactions de séquençage. Les réactions de séquençage sont symbolisées par le bloc 4 sur la figure 1.

Le marquage des chaînes obtenues, symbolisé par le bloc 6, peut être réalisé en utilisant un isotope ou une combinaison d'isotopes stables, constituants éventuels de petites molécules stables, afin de marquer spécifiquement les chaînes issues de chacune des quatre séries de réactions chimiques.

Le marquage isotopique selon l'invention des chaînes d'ADN peut être réalisé au niveau de l'un des trois composés chimiques constituant les nucléotides, c'est-à-dire la base, le sucre ou le groupement triphosphate ou éventuellement sur un groupement alpha-thiophosphaté dans lequel l'atome d'oxygène lié au premier phosphore est remplacé par un atome de soufre.

Le marquage des chaînes peut être réalisé de façon intrinsèque sur le carbone, l'hydrogène, l'azote, l'oxygène ou même le soufre entrant dans la constitution des trois composés chimiques d'un nucléotide. L'utilisation d'isotopes naturels stables, selon l'invention, offre un choix plus important que les isotopes radioactifs généralement utilisés ($^{32}P$, $^{35}S$, $1^{14}C$, $^{3}H$) pouvant entrer dans la constitution des différents composés nucléotidiques.

Une liste de ces isotopes stables, incluant leur abondance naturelle relative, est donnée dans le tableau 1.

Tableau 1

Isotopes naturels stables pour marquage intrinsèque des composés nucléotiques

| Isotopes | Abondance relative (%) |
|---|---|
| D | 0,015 |
| $^{13}C$ | 1,10 |
| $^{15}N$ | 0,37 |
| $^{17}O$ | 0,038 |
| $^{18}O$ | 0,20 |
| $^{32}S$ | 95,02 |
| $^{33}S$ | 0,75 |
| $^{34}S$ | 4,21 |
| $^{36}S$ | 0,02 |

Du point de vue chimique, les réactions pour incorporer un isotope stable ou un isotope radioactif sont les mêmes.

Les divers procédés de marquage de molécules biologiques par des isotopes stables existent et sont largement répandus (voir à cet effet l'article "The preparation of purines, pyrimidines, nucleotides and related compounds labelled with $^{17}O$, $^{18}O$, and other stable isotopes" E. Petreanu, J.S. Cohen and D. Samuel. Proceedings of the 2nd International Conference on methods of preparing and storing labelled compounds, pp. 489-497). Différentes molécules marquées, en particulier les nucléotides peuvent être produites par voie de synthèse ou par voie enzymatique. En particulier, de nombreux composés nucléotidiques marqués avec des isotopes stables sont disponibles sur le marché pour des applications en biologie et en pharmacologie différentes de celles de l'invention (voir par exemple l'article "Dehalogenation Reactions in Fast Atom Bombardment Mass Spectrometry", Anal. Chem. 1984.56. 1975.77)

Des exemples de nucléotides marqués à l'aide d'isotopes naturels sont donnés dans les annexes I et II.

Ces exemples ont uniquement pour but d'illustrer des moyens de marquage possibles utilisables dans l'invention.

Comme données dans ces annexes I et II, le marquage peut s'étendre à toutes les formes de nucléotides et en particulier aux formes désoxytriphosphate et didésoxytriphosphate (annexe I) ainsi qu'à leurs analogues alpha-thiotriphosphate (annexe II). Ceci autorise le marquage des différentes sortes de chaînes d'ADN issues des divers types de réaction de séquençage telles que celles issues des méthodes de Sanger ou de Maxam-Gilbert décrites précédemment.

Comme autre nucléotide marqué intrinsèquement, on peut citer le 1 ou 3-$^{15}$NdATP, l'α-$^{18}$OdCTP, le 4-$^{18}$OdTTP, le 2-$^{13}$CdATP ou dCTP, le 8-$^{13}$CdATP, le 5'-$^{13}$CdATP, l'α-SdATP, l'α-SdGTP, l'α-SdCTP, l'α-SdTTP où S est l'isotope 32, 34, 33 ou 36, le méthyl $^{13}$CdTTP.

Il est aussi possible, selon l'invention, d'effectuer un marquage extrinsèque des nucléotides. Ce type de marquage comporte l'avantage de pouvoir lier au nucléotide des atomes (sous forme mono ou polyatomique) qui ne sont pas naturellement présents dans ces molécules. Ces marqueurs sont constitués d'éléments ou de petits complexes moléculaires d'éléments alcalins, alcalino-terreux ou halogéniques.

Dans le cadre des réactions de séquençage, le marquage extrinsèque selon l'invention présente moins de risque de dénaturer les propriétés physiques et chimiques des nucléotides, contrairement à l'adjonction selon l'art antérieur de grosses molécules telles que les chromophores ou fluorophores qui ne peuvent être liées que par l'intermédiaire de bras spécifiques relativement complexes. Ainsi, les éléments ou petits complexes moléculaires selon l'invention peuvent être directement greffés au nucléotide en profitant des liaisons covalentes disponibles au sein de la base ou du sucre de chaque nucléotide (voir par exemple l'article d'Anal. Chem. cité précédemment).

Des exemples de marquage extrinsèques selon l'invention sont donnés en annexe III. Cet exemple donne un jeu complet de bases nucléotidiques de l'ARN marquées extrinsèquement. En particulier, X représente un atome de fluor, de chlore, de brome ou d'iode.

Comme nucléotide marqué par des halogènes utilisables dans l'invention, on peut citer le 8-bromo dGTP, le 5-bromo dCTP, le 3′-bromo, chloro ou iodo dTTP, le 5-bromo iodo ou fluoro dTTP, le 3′-FdGTP.

En tenant compte de l'existence de plusieurs isotopes stables possibles des différents marqueurs extrinsèques (éléments halogènes par exemple), les possibilités de marquage isotopiques des nucléotides sont fortement accrues.

Une liste des différents isotopes stables d'éléments halogènes est présentée dans le tableau 2, accompagnés de leur abondance naturelle relative.

Tableau 2

Isotopes naturels stables des éléments halogènes pour le marquage extrinsèque

| Isotopes | Abondance relative (%) |
|---|---|
| $^{19}F$ | 100 |
| $^{35}Cl$ | 75,77 |
| $^{37}Cl$ | 24,23 |
| $^{79}Br$ | 50,69 |
| $^{81}Br$ | 49,31 |
| $^{127}I$ | 100 |

Conformément à l'invention, il est possible d'effectuer des marquages multiples des nucléotides des chaînes issues des réactions de séquençage, ces marquages étant des marquages intrinsèques et/ou extrinsèques.

En premier lieu, on peut citer des possibilités d'incorporer plusieurs isotopes marqueurs de même type sur un nucléotide donné. Comme nucléotide marqué de ce type, on peut citer par exemple le 1,3-$^{15}$N dATP, le 1,3-$^{15}$NdCTP, le 2,8-dichloro ou dibromo ou difluoro dATP, le 5,5-dibromo dCTP. Ceci permet d'accroître d'autant la sensibilité obtenue lors de la détection et de l'identification des chaînes nucléotidiques.

En second lieu, plusieurs marqueurs différents peuvent être incorporés au sein d'un même nucléotide afin d'affiner sa signature. De plus, la détection simultanée de plusieurs marqueurs augmente considérablement les combinaisons de marquages possibles.

L'exemple suivant montre que trois types de marqueurs isotopiques stables suffisent à l'identification de quatre nucléotides différents :

| | |
|---|---|
| $^{13}C$ seul | Nucléotide de type A |
| $^{13}C$ + $^{17}O$ | Nucléotide de type G |
| $^{13}C$ + $^{15}N$ | Nucléotide de type C |
| $^{15}N$ + $^{17}O$ | Nucléotide de type T |

Ainsi, les marquages intrinsèques et extrinsèques des nucléotides à l'aide d'isotopes stables peuvent être combinés et offrent de réelles possibilités multiples et variées.

Cette méthode de marquage s'avère plus souple et plus riche d'évolution que les marquages radio-isotopiques ou par fluorophores selon l'art antérieur qui restent forcément plus limités dans leurs possibilités. De plus, la plupart des inconvénients rencontrés avec ces derniers lors de leur manipulation et de leur utilisation sont évités voire complètement éliminés.

Les chaînes d'ADN marquées sont alors placées dans une source 8 appropriée pour leur séparation par chromatographie, représentée par le bloc 10.

A cet effet, les chaînes marquées sont introduites dans une colonne de chromatographie ou colonne d'électrophorèse sur gel en vue de les séparer suivant leur taille, leur charge ou même d'autres propriétés physiques. Pour le séquençage d'ADN, les chaînes marquées dont la longueur diffère d'un simple nucléotide sont séparées par électrophorèse dans une matrice de gel polyacrylamide.

Il en résulte que dans le fond et près du fond de la colonne de chromatographie, les chaînes d'ADN sont résolues les unes des autres et peuvent être extraites et prises en compte par une source d'ions en vue

d'ioniser les chaînes séparées, comme indiqué par le bloc 12.

Ensuite les ions sont sélectionnés et détectés par spectrométrie de masse comme indiqué en 14 puis les signaux fournis par la spectrométrie de masse sont traités, comme indiqué en 16, en vue de reconstituer l'ADN de départ.

L'installation permettant le séquençage automatisé de l'ADN, conformément à l'invention, est schématisée sur la figure 2. La fragmentation du génome, les réactions chimiques de séquençage et le marquage étant effectués de façon discrète ou de façon indépendante dans le temps, ne sont pas représentés sur la figure 2.

L'installation représentée comporte une source 18 de chaînes marquées d'ADN à l'aide d'isotopes stables selon l'invention contenant une cuve 20 destinée à recevoir l'échantillon 21 des chaînes marquées d'ADN mises en solution dans un tampon portée à la haute tension grâce à une source d'alimentation électrique 22.

Le fond de la cuve 20 est muni d'un orifice couplé à un tube électrophorétique 24 de 1 mm de diamètre environ monopiste pouvant contenir un gel polyacrylamide ; l'extrémité de sortie 26 du tube constituant une anode débouche à l'intérieur d'une source d'ions 28 portée au potentiel de la masse.

Grâce à un jet de gaz rare et en particulier d'argon, introduit dans la source 28, via une conduite 30, au sein du plasma créé électromagnétiquement, les chaînes marquées issues du tube électrophorétique 24 sont pulvérisées sous forme d'aérosols.

Différentes sources d'ions adaptées au couplage de différents types de tube électrophorétique peuvent être utilisées. Elles font appel ou peuvent combiner divers processus physiques courants d'atomisation tels que l'électrospray, le thermospray, la nébulisation.

Les aérosols obtenus sont alors introduits dans une source à plasma 32 équipée de bobines électromagnétiques 33 reliées à un générateur radiofréquence 34. Dans la zone 32a la plus chaude du plasma, où la température atteint 7000 à 8000° K, les molécules d'ADN sont atomisées et ionisées.

La torche à plasma est la source préférée d'ionisation selon l'invention car elle permet l'atomisation complète des molécules et induit une simplicité maximale dans le spectre de masse obtenu après analyse d'éléments simples.

Les ions formés sont alors pris en charge par l'optique d'entrée 36 d'un spectromètre de masse 38 qui peut être du type secteur magnétique où un vide différentiel s'installe grâce à des pompes primaires et secondaires 39 ; la pression au niveau de l'optique ionique 36 est de l'ordre de $10^{-4}$ torr ($10^{-2}$Pa) et la pression au niveau des pièces polaires 40 du spectromètre est de l'ordre de $10^{-6}$ torr ($10^{-4}$ Pa).

Le premier rôle du spectromètre de masse 38 est de sélectionner et d'identifier grâce à son optique ionique 36 et ses pièces polaires 40, la présence des masses spécifiques obtenues grâce au traceur isotopique utilisé précédemment. Le spectromètre de masse 38 peut opérer en mode sélectif ce qui consiste à balayer uniquement les masses caractéristiques dues à la présence du marqueur isotopique, afin de fournir un maximum de sensibilité.

La détection par spectrométrie de masse permet à la fois de détecter les chaînes d'ADN marquées au fur et à mesure de leur sortie de la colonne chromatographique 24 et d'identifier grâce à leur marqueur spécifique à quel nucléotide elles correspondent. Cette information donne la séquence d'ADN.

Les molécules ionisées et sélectionnées par le spectromètre de masse sont alors détectées à l'aide d'un détecteur 42 situé à la sortie du spectromètre 38, du type multiplicateur d'électrons et qui est sensible à l'impact d'un seul ion. Les ions sélectionnés peuvent donc être détectés individuellement ce qui favorise l'obtention d'une sensibilité maximale de l'installation totale de séquençage.

Le couplage en ligne d'une source 18 de chaînes marquées, du tube d'électrophorétique 24, de la source d'ions 28 équipée de la torche à plasma 32, et d'un spectromètre de masse 38 équipé de son optique d'entrée 36, de ses pièces polaires 40 et de son détecteur 42 sont en particulier donnés dans l'article Analytical Chemistry, vol. 59, n° 8 d'avril 1987, p. 1230 1232, intitulé "On-line mass spectrometric detection for capillary zone electrophoresis" ou dans l'article Analytical Chemistry, vol. 52, N° 14 de Décembre 80, p. 2283-2289, intitulé "Inductively coupled argon plasma as an ion source for mass spectrometric determination of trace elements".

Le signal électrique issu du détecteur 42 est alors amplifié grâce à l'amplificateur 44 avant d'être reçu dans un micro-ordinateur 46 du type PC qui gère automatiquement, à la fois le pilotage des divers sous-ensembles (18, 28, 38) de l'installation, le traitement et la représentation des données.

En fonction du temps, un signal proportionnel au nombre de coups reçus par unité de temps par le détecteur 42 est mesuré pour chacune des masses caractéristiques des marqueurs isotopiques utilisés. Cette information indique le nucléotide qui caractérise une chaîne d'ADN analysée dont la longueur est particulière à la fenêtre temporelle d'observation donnée.

La figure 3 représente le type de données obtenues par l'installation représentée sur la figure 2. A titre explicatif, la ligne $A_1$ correspond à une chaîne marquée de n nucléotides, dont l'une des extrémités se termine par l'adénine ; les lignes $A_2$ et $A_3$ correspondent, respectivement, à des chaînes marquées de n-4 et n-9 nucléotides dont l'une des extrémités se termine par l'adénine ; les trois adénines terminales étant marquées par isotope stable, appelé isotope 1. Il en est de même pour les autres bases.

L'extrême sensibilité de l'ensemble du système de détection de l'invention (inférieure au ppb) indique que ce procédé est tout à fait adéquat pour effectuer une analyse de séquence d'ADN.

A titre illustratif, on donne ci-après, un exemple de séquençage d'un ADN hypothétique, en se référant à la figure 3.

L'ADN à séquencer est le TGACTGCACGTA. Aussi, toutes les chaînes marquées dans la source de

chaînes 8 ou 18 commencent par un T et se terminent par l'une des quatre bases X. Ces chaînes résultent des réactions chimiques de séquençage de Sanger ou Maxam/Gilbert. Les chaînes sont présentes pêle-mêle dans l'échantillon 21. Elles comportent de 1 à 12 nucléotides tels que : TGACTGCACGTA, TGACTGCA, TGA, TGAC, TGACT, T, TGACTGCAC.

Ces chaînes sont séparées selon leur taille par électrophorèse (étape 10) dans un tube 24 monopiste. On obtient les bandes d'électrophorèse suivantes ordonnées de gauche à droite :

TTT ... T ... TTT
GGG ... G ... GG
AAA ... A ... A
CCC ... C
TTT ... T ...
GGG ... G
CCC ...
AAA
CCC
GGG
TT
A

Chaque colonne représente une bande d'électrophorèse qui contient en fait de l'ordre de plusieurs dizaines de millions d'exemplaires de chacune des chaînes.

Ces chaînes sont ensuite atomisées puis ionisées au fur et à mesure de leur sortie du gel électrophorétique, dans la source d'ions 28 (étape 12).

Les chaînes TG, TGACTG, etc, se terminant par de la guanine sont atomisées et ionisées et fournissent un marqueur *G de type G. De même, les chaînes se terminant par de l'adénine, telles que TGA, TGACTGCA, etc, sont atomisées et ionisées et fournissent des marqueurs *A, de type A. De même, les chaînes se terminant respectivement par C et T fournissent des marqueurs *C et *T. Ces marqueurs se trouvent mêlés à d'autres ions et, en particulier, à des ions de carbone, azote, oxygène ou hydrogène provenant de l'ionisation des chaines, soit :

O N
G*
H C

Aussi, le spectromètre de masse 38 (étape 14) permet la sélection des marqueurs puis leur détection ion par ion.

La détection, au cours du temps, d'ions caractéristiques des marqueurs utilisés permet à la fois de déceler la présence des chaînes d'ADN prises en compte par la source d'ions, et d'identifier leur famille suivant le type d'ions marquant leur extrémité. L'ordre de ces marqueurs pris en compte par l'ordinateur 46 (étape 16) donne l'ordre des bases ou séquences de l'ADN, soit ici GGAC, etc...

L'invention ayant été décrite en détail, il est évident que de l'ARN ou d'autres types de molécules biologiques complexes peuvent être analysées grâce au procédé et à l'installation selon l'invention : l'électrophorèse a en effet d'autres applications que le séquençage de l'ADN.

Cette technique séparative sert aussi à séparer des fragments de molécules biologiques complexes (ADN, ARN, protéines) afin de déterminer leur taille. Pour se faire, la vitesse de migration de ces fragments est comparée à celle de fragments de tailles connues, les échantillons étant déposés sur des pistes adjacentes. Grâce à l'invention, il est possible de marquer avec des isotopes ou petites molécules stables différentes, les différents échantillons ainsi que les marqueurs de taille. Ceux-ci sont ensuite déposés sur une même piste d'électrophorèse puis introduits en ligne dans la source du spectromètre de masse. L'atomisation des fragments permet d'isoler le marqueur et de le détecter. Le type de marqueur détecté indique l'appartenance du fragment à tel ou tel échantillon et son instant d'arrivée, par rapport aux instants d'arrivée des standards, indique sa taille.

De plus, ce type de marquage utilisé pour marquer certains acides aminés d'une protéine permet de détecter soit la protéine au sein d'un mélange de protéine, soit la présence de ces acides aminés au sein de cette protéine.

ANNEXE I

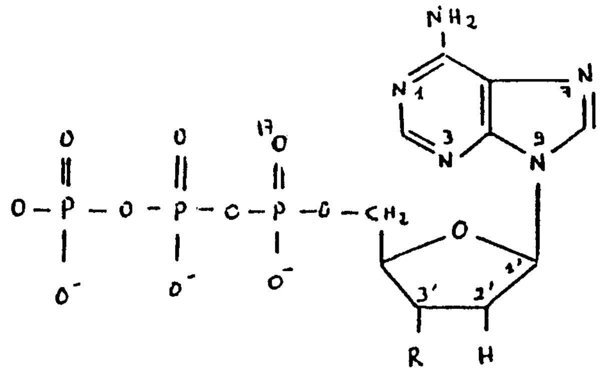

α- $^{17}$O (d) dATP

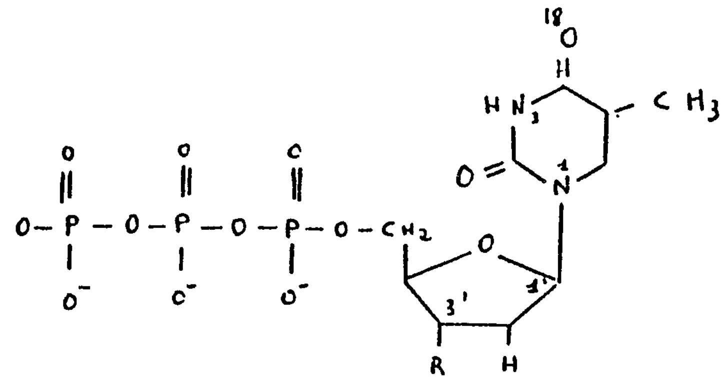

4- $^{18}$O (d) dTTP

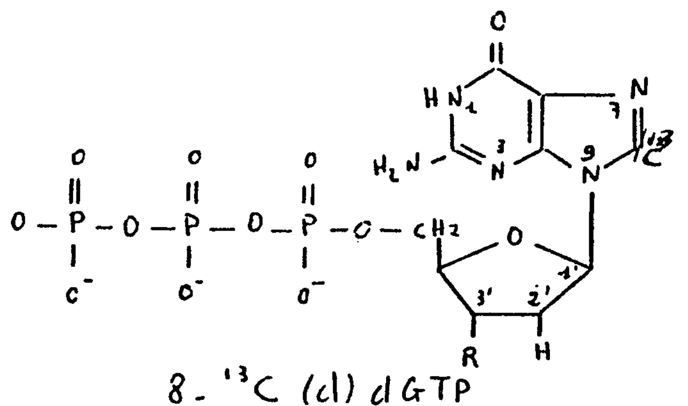

8- $^{13}$C (d) dGTP

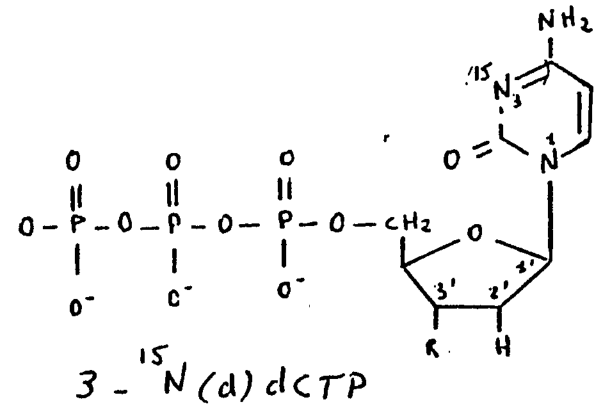

3 - $^{15}$N (d) dCTP

R = OH

R = H

ANNEXE II

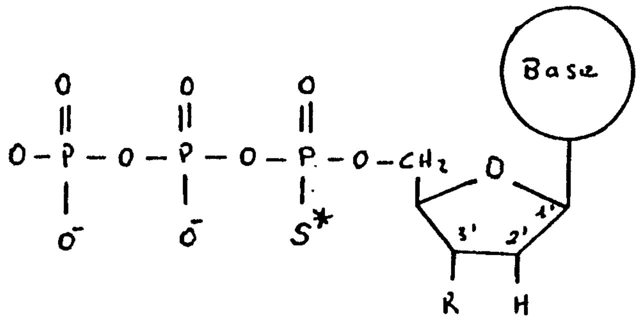

R = H ou OH

alpha - thio (d) dNTP

S* = $^{32}S$, $^{33}S$, $^{34}S$, $^{36}S$

ANNEXE III

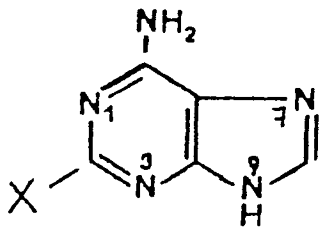

ADENINE

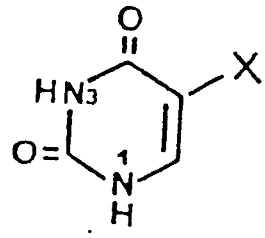

URACIL

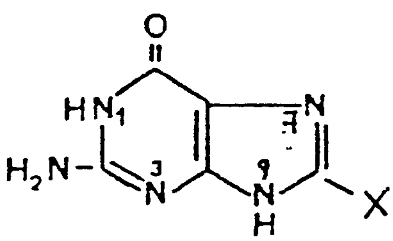

GUANINE

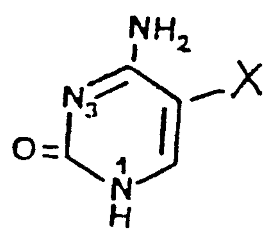

CYTOSINE

**Revendications**

1. Procédé d'identification de la séquence des bases d'un acide nucléique comportant essentiellement :

a) une étape (2) de production de fragments de l'acide nucléique à séquencer,

b) une étape (4) de réactions chimiques spécifiques desdites bases pour former des familles de chaînes de tailles différentes dont l'une des extrémités est constituée d'une base spécifique,

c) une étape (6) de marquage spécifique desdites chaînes avec des isotopes ou de petites molécules stables,

d) une étape (10) de séparation des chaînes marquées,

e) une étape d'atomisation et d'ionisation (12) de chaque chaîne marquée puis,

f) une étape de détection (14) en temps réel des ions formés par spectrométrie de masse.

2. Procédé selon la revendication 1, caractérisé en ce que le marquage est réalisé de façon intrinsèque avec au moins un isotope choisi parmi D, $^{13}C$, $^{15}N$, $^{17}O$, $^{18}O$, $^{32}S$, $^{33}S$, $^{34}S$ et $^{36}S$.

3. Procédé selon la revendication 1 ou 2, caractérisé en ce que le marquage est réalisé de façon

extrinsèque avec au moins un isotope choisi parmi $^{19}F$, $^{35}Cl$, $^{37}Cl$, $^{79}Br$, $^{81}Br$ et $^{127}I$.

4. Procédé selon l'une quelconque des revendications 1 à 3, caractérisé en ce que l'on sépare les chaînes par chromatographie avec une seule piste chromatographique.

5. Procédé selon l'une quelconque des revendications 1 à 4, caractérisé en ce que l'acide nucléique est l'ADN.

6. Procédé selon la revendication 5, caractérisé en ce que le marquage est effectué sur les bases, les sucres ou les triphosphates des formes desoxy-triphosphatées (dXTP) ou didésoxy-triphosphatées (ddXTP) ou de leurs analogues alpha-thiotriophosphatées.

7. Installation pour l'identification de la séquence des bases d'un acide nucléique comprenant :
- une source (18) de chaînes de nucléotides de tailles différentes, marquées par des isotopes ou de petites molécules stables,
- des moyens de séparation (20, 22, 24, 26) de ces chaînes marquées,
- des moyens (28, 30, 32) d'atomisation et d'ionisation des chaînes sortant des moyens de séparation,
- un spectromètre de masse (38) pour sélectionner et détecter les ions, et
- des moyens de traitement (44, 46) des signaux fournis par le spectromètre.

8. Installation selon la revendication 7, caractérisée en ce que les moyens d'ionisation comprennent une torche à plasma (32).

9. Installation selon la revendication 7 ou 8, caractérisée en ce que les moyens de séparation comportent un tube électrophorétique (24) monopiste et des moyens (20, 22, 26) pour assurer le passage des chaînes dans le tube.

10. Procédé d'identification d'une protéine dans un mélange de protéines ou de certains de ses acides aminés, comportant essentiellement :
- une étape de marquage spécifique desdits acides aminés avec des isotopes ou de petites molécules stables,
- une étape de séparation des protéines marquées,
- une étape d'atomisation et d'ionisation de chaque protéine marquée puis,
- une étape de détection en temps réel des ions formés par spectrométrie de masse.

11. Procédé d'identification de la taille des fragments de molécules biologiques complexes, comprenant essentiellement :
- une étape de marquage des fragments,
- une étape de séparation selon leur taille, des fragments marqués spécifiquement et mêlés à des standards de taille,
- une étape d'atomisation et d'ionisation des fragments puis une détection en temps réel des ions par spectrométrie de masse.

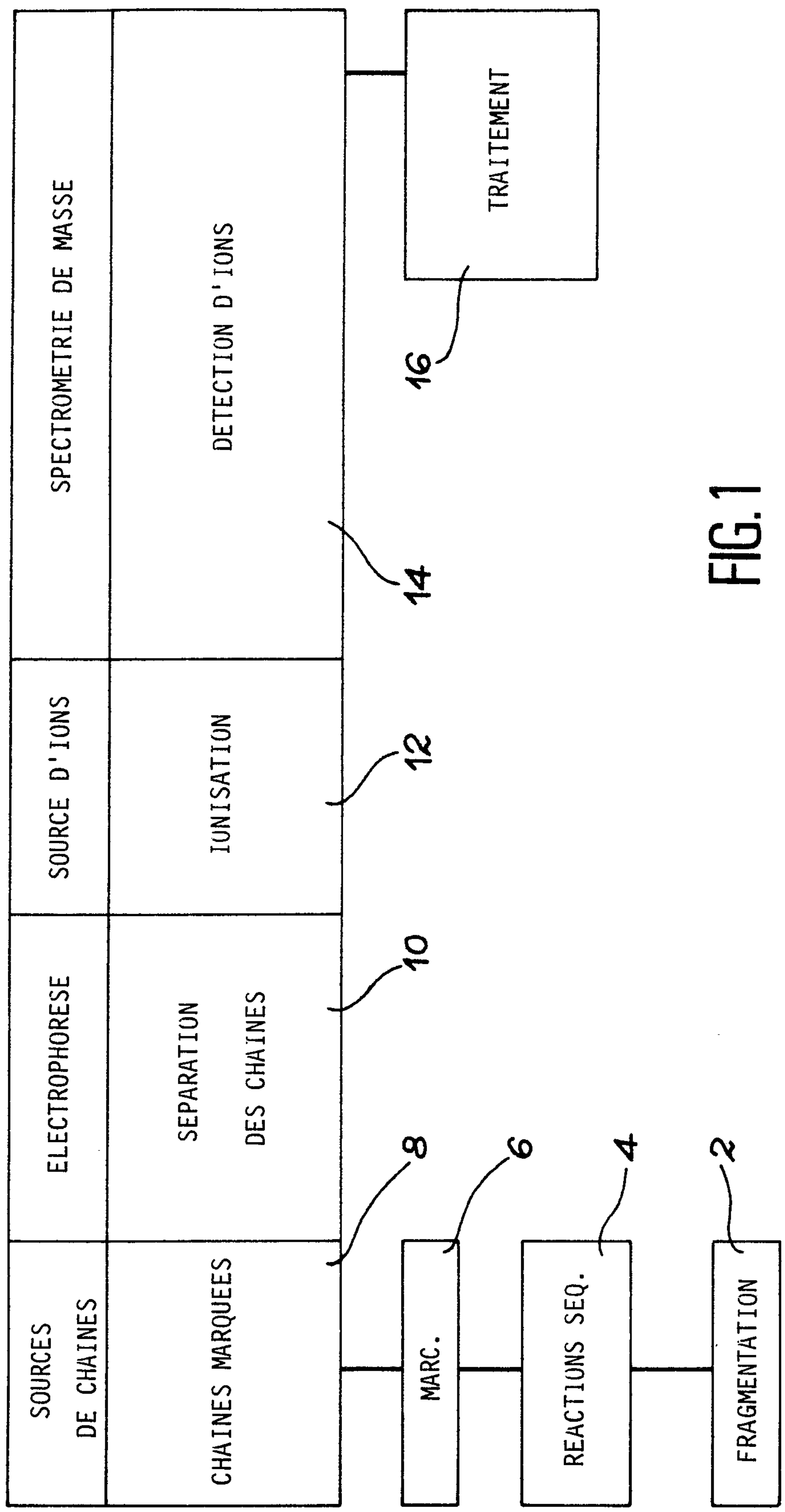
SOURCES DE CHAINES
CHAINES MARQUEES
8
ELECTROPHORESE
SEPARATION DES CHAINES
10
SOURCE D'IONS
IONISATION
12
SPECTROMETRIE DE MASSE
DETECTION D'IONS
14
TRAITEMENT
16
MARC.
6
REACTIONS SEQ.
4
FRAGMENTATION
2

FIG. 1

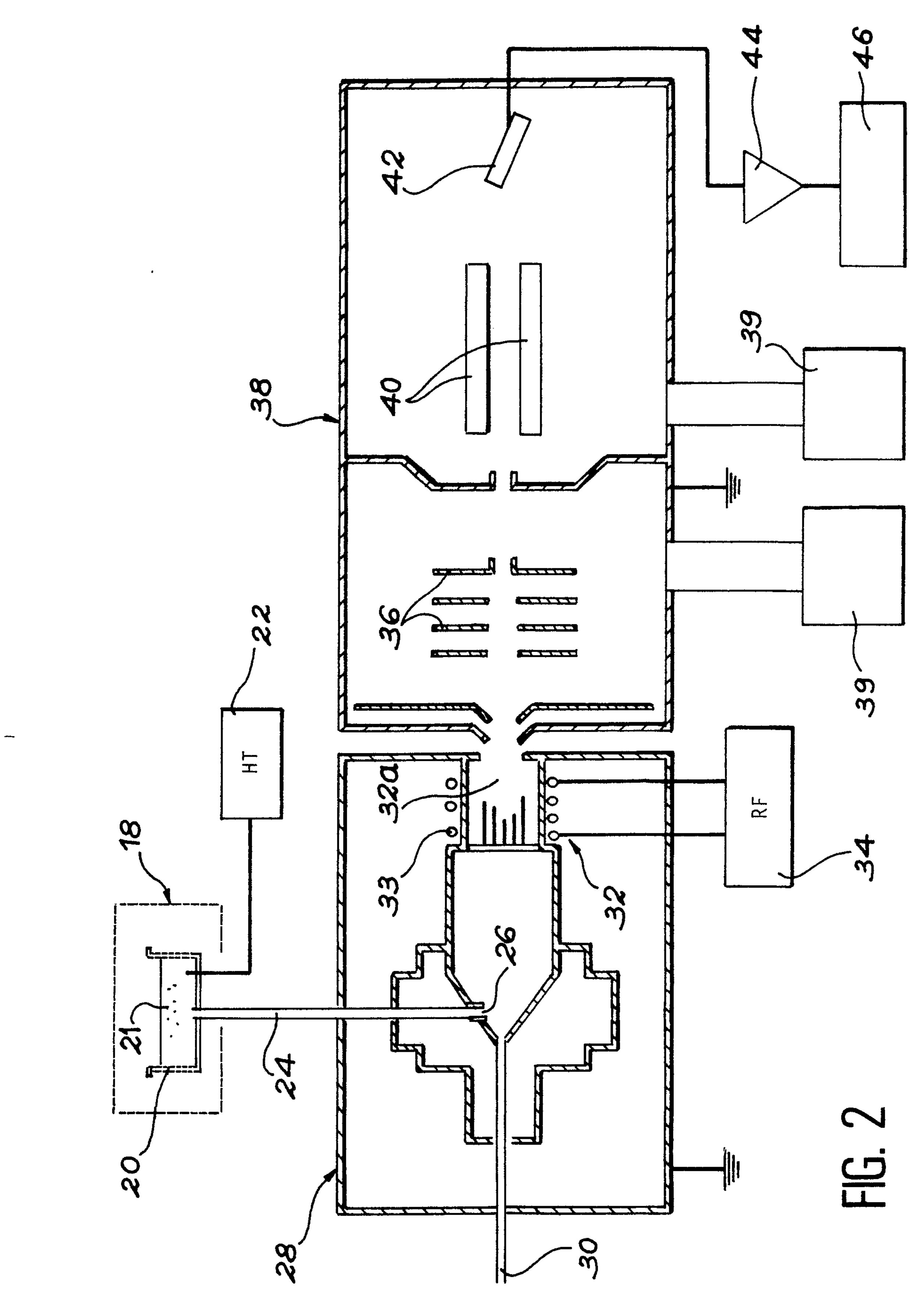


FIG. 2

1) DIAGRAMME IDEALISE DE BANDES D'ADN MARQUEES PAR DES ISOTOPES STABLES DANS LE GEL D'UN TUBE ELECTROPHORETIQUE

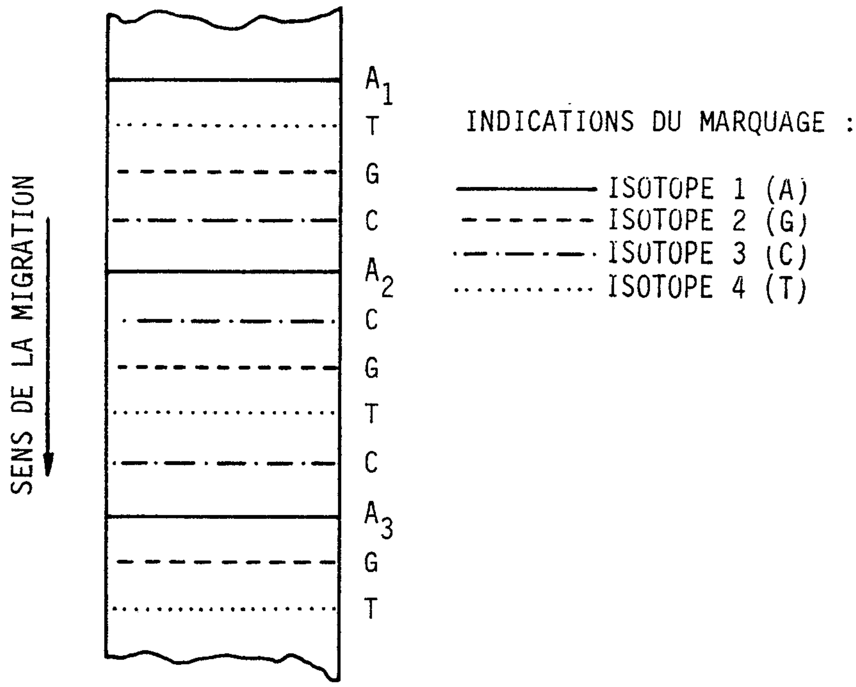


2) RESULTAT IDEALISE DE LA DETECTION DES BANDES MARQUEES A PARTIR DE LA REPRESENTATION DU TUBE CI-DESSUS

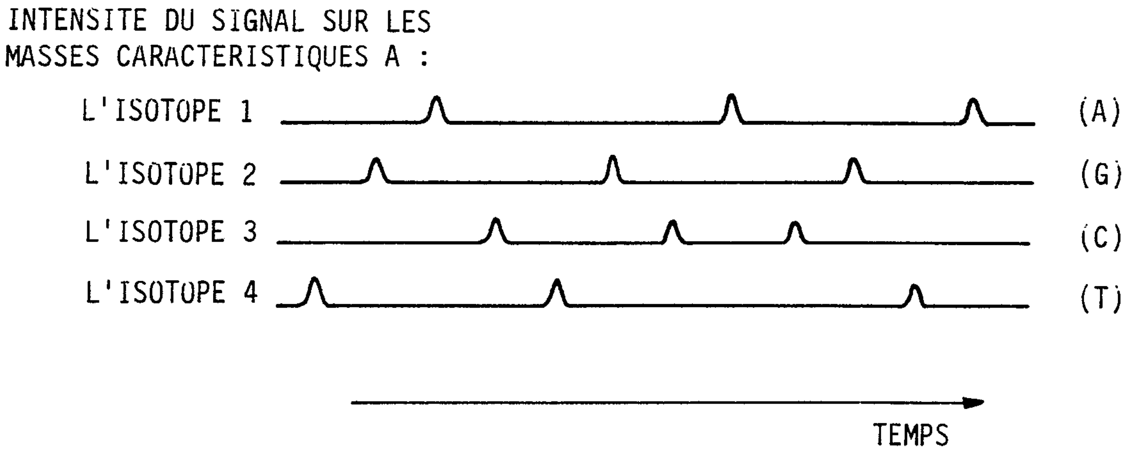


FIG. 3

Office européen des brevets

# RAPPORT DE RECHERCHE EUROPEENNE

Numero de la demande

EP 89 40 2548

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | FR-A-2 558 262 (CALIFORNIA INSTITUTE OF TECHNOLOGY)<br>* En entier * | 1-11 | C 12 Q 1/68 |
| Y | DE-A-3 312 929 (GESELLSCHAFT FÜR BIOTECHNOLOGISCHE FORSCHUNG mbH)<br>* En entier * | 1-11 | |
| Y | US-A-4 022 876 (M. ANBAR)<br>* En entier * | 1-11 | |
| A | WO-A-8 505 642 (K. RODLAND) | | |
| A | PATENT ABSTRACTS OF JAPAN, vol. 10, no. 111 (P-451)[2168], 25 mai 1986; & JP-A-60 242 368 (HITACHI SEISAKUSHO K.K.) 02-12-1985 | | |

DOMAINES TECHNIQUES RECHERCHES (Int. Cl.5)

C 12 Q
G 01 N

Le présent rapport a été établi pour toutes les revendications

| Lieu de la recherche | Date d'achèvement de la recherche | Examinateur |
|---|---|---|
| LA HAYE | 21-11-1989 | CARTAGENA Y ABELLA P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)